# EUROPEAN PATENT SPECIFICATION

(11) **EP 4 100 090 B1**
(45) Date of publication and mention of the grant of the patent: **10.09.2025**
(21) Application number: 20917754.2
(22) Date of filing: 05.02.2020
(51) Int. Cl.: A61M 16/04, A61J 15/00, A61M 25/00, A61M 39/00, A61M 11/00, A61M 16/08, A61M 16/10

(54) **MULTI-TUBE ADAPTER**
ADAPTER FÜR MEHRERE SCHLÄUCHE
ADAPTATEUR MULTI-TUBE

(43) Date of publication of application: 14.12.2022
(73) Proprietor: JOYEUX LAB COMPANY LIMITED, Da'an Dist 106 Taipei City (CN)
(72) Inventor: LIU, Yi-Ching, Taipei City, 104 (TW); TSAI, Hsin-Hua, Taipei City, 105 (TW)
(74) Representative: Lang, Christian
(86) International application number: PCT/US2020/016830
(87) International publication number: WO 2021/158216

(56) References cited:
- WO-A1-2011/057458
- WO-A1-2014/166136
- WO-A1-2017/184843
- WO-A1-2019/153803
- CN-A- 102 921 083
- US-A- 4 176 660
- US-A- 5 499 625
- US-A1- 2010 147 296
- US-A1- 2015 297 847
- US-A1- 2017 209 022

## Description

### FIELD OF THE INVENTION

The present disclosure relates to medical devices, particularly, to a multi-tube adapter used for application to the digestive system and/or respiratory system of a human body.

### BACKGROUND OF THE INVENTION

Patients with eating or swallowing difficulties generally need a catheter inserted through the nasal cavity, throat and esophagus into the stomach to facilitate input of liquid food or reversely output of the stomach contents for the purpose of relieving stress or medical diagnosis.

Patients with productive cough (i.e. cough with sputum) difficulties generally need a catheter entered through the nasal or oral cavity into the throat and the upper trachea to discharge the sputum by means of negative pressure suction, in order to keep the respiratory tract open.

Patients with hypoxemia need a catheter to deliver high concentration oxygen to the nasal cavity to improve respiratory performance and the oxygen concentration in blood.

Patients with the above needs simultaneously require multiple catheter placements to maintain life supporting functions such as eating, expectoration and breathing.

Document WO 2017/184843 A1 discloses an enteral feeding catheter with one or more environmental sensors for automated delivery of liquid food or drugs. A conventional multi-tube adapter as defined by the preamble of claim 1 is basically known therefrom.

Nonetheless, the present disclosure provides a multi-tube adapter to provide a new way of caretaking capable of overcoming the shortcomings of the prior art.

### SUMMARY OF THE INVENTION

The first objective of the present disclosure is to provide a multi-tube adapter connected to one or a plurality of outer tubes to establish multiple supply paths, thereby achieving the purpose of supporting human internal organs or discharging secretions (e.g. sputum, saliva, etc.) from inside the human body.

Said multi-tube adapter is capable of being independently used in the digestive system or the respiratory system or in both systems simultaneously.

Said multi-tube adapter has a multi-chamber housing where the chambers are not connected with each other, in order to achieve the purpose of preventing different substances (e.g. liquid, medicinal powder, gas, etc.) among the chambers from interfering with one another.

Said multi-tube adapter has a plurality of external ends (i.e. multiple connection ports) for respectively connecting to the outer tubes and a single internal end (i.e. the extension as mentioned in the present disclosure).

In said multi-tube adapter the external ends and the internal end are capable of exchanging substances unidirectionally or bidirectionally to achieve the purpose of a single multifunctional device.

For said multi-tube adapter the purpose of avoiding misplacement of the outer tubes may be achieved by means of an anti-tube misplacement (or foolproof) mechanism.

For said multi-tube adapter an adaptation to various functionalities may be achieved by means of adjusting the dimensions of the chambers.

Said multi-tube adapter further has a closing component to close the supply path to protect the unused supply paths.

For said multi-tube adapter the disposition thereof on a patient's nose, face, ears or head may be facilitated by means of a fastener fixing the outer tube.

Said multi-tube adapter may be further provided in combination with electronic components (e.g. control module, sensor, etc.) to achieve the purpose of monitoring, calculating and recording.

Said multi-tube adapter may be further provided with an indicator disposed at the opening of the connection port for the purpose of indicating the supply path.

Said multi-tube adapter has an extension with a plurality of openings (i.e. providing multiple openings within the caliber of a single tube body) where the length of the extension may be flexibly determined so as to be nearby or inside an organ.

The extension of said multi-tube adapter may be alternatively connected to an inner tube through which the extension may be disposed nearby or inside an organ.

The opening of the extension may be disposed nearby an organ in order to achieve the purpose of directly supplying or extracting (or discharging) substances from the organ or conducting drainage.

The multi-tube adapter has a path enabling insertion of an external diagnostic device to achieve the purpose of diagnosing and observing an organ.

For said multi-tube adapter the outer tube and/or inner tube may be respectively replaced to adapt to various needs as well as achieving efficacies such as cleansing, reducing costs, etc.

In order to achieve the aforesaid objectives among others, the present invention provides a multi-tube adapter according to claim 1, which is connected to a plurality of outer tubes for application to a digestive system and a respiratory system of a human body. The multi-tube adapter includes a housing. The housing forms an accommodating space including a first chamber and a second chamber. The first chamber and the second chamber are not connected with each other. Furthermore, the first chamber has a first connection port while the second chamber has a second connection port, and the first chamber and the second chamber have an extension with a first opening and a second opening. The first opening is connected to the first chamber while the second opening is connected to the second chamber. Therein, the first connection port is connected to the first chamber and the first opening, enabling the first connection port to further establish a first supply path with the respiratory system via the first chamber and the first opening; the second connection port is connected to the second chamber and the second opening, enabling the second connection port to further establish a second supply path via the second chamber and the second opening. The multi-tube adapter further includes a closing component coupled to at least one of the first connection port and the second connection port for selectively closing the first supply path and the second supply path.

Compared to prior art, the multi-tube adapter disclosed in the present invention may be connected to a plurality of outer tubes which provide various functions (e.g. feeding food, supplying oxygen, drawing sputum, injecting medicine) by means of connecting to an external equipment. As the multi-tube adapter disclosed in the present invention provides a plurality of independent chambers, the chambers are capable of executing designated functions independently without interference. Moreover, an extension which is a single tube body connected to multiple chambers is provided in the present invention, and the single tube body provides multiple openings disposed respectively nearby or inside an organ (e.g. stomach, lung, etc.)

In another embodiment, a plurality of foolproof mechanisms such as structure differentiation (e.g. tube diameter, tube shape, etc.), identity differentiation (e.g. color, text, pattern, etc.) are provided to avoid misplacement of the tubes which may cause damage to a patient's organ or endanger his/her life.

In yet another embodiment, by flexibly adjusting the dimensions of the openings of the chamber and/or extension, different needs may be addressed to, for example, the dimensions of an opening required by gas is smaller than that required by liquid.

In another embodiment, a sensor or a control module is provided, to intellectualize the multi-tube adapter for use in monitoring, recording, displaying the status related to each chamber of the multi-tube adapter.

### BRIEF DESCRIPTION OF THE DRAWINGS

FIG.1 is a sectional structure diagram of a first embodiment of the multi-tube adapter of the present invention.
FIG.2 is a schematic diagram of the first embodiment of the multi-tube adapter of the present invention being connected to an outer tube.
FIG.3 is a structure diagram of a second embodiment of the multi-tube adapter of the present disclosure.
FIG.4 is a structure diagram of a third embodiment of the multi-tube adapter of the present disclosure.
FIG.5 is a structure diagram of a fourth embodiment of the multi-tube adapter of the present disclosure.
FIG.6 is a structure diagram of a fifth embodiment of the multi-tube adapter of the present disclosure.
FIG.7 is a structure diagram of a sixth embodiment of the multi-tube adapter of the present disclosure.

### DETAILED DESCRIPTION OF THE PREFERRED EMBODIMENTS

In order to fully understand the objectives, features, and functions of the present disclosure, the present disclosure is described in detail as follows by the following specific embodiments along with the accompanying figures.

In the present disclosure, "a" or "one" is used to describe the units, elements, and components described herein. This is done for the convenience of description only and provides a general meaning to the scope of the present disclosure. Therefore, unless stated otherwise, this description should be understood to include one, at least one, and more than one.

In the context of the present disclosure, the terms "comprising", "including", "having" or "containing" are intended to encompass non-exclusive inclusions. For example, an element, structure, article, or device that comprises a plurality of elements is not limited to such elements as listed herein, but may include those not specifically listed but which are generally inherent in the element, structure, article, or device. In addition, the term "or" is used to mean an inclusive "or" rather than an exclusive "or" unless expressly stated to the contrary.

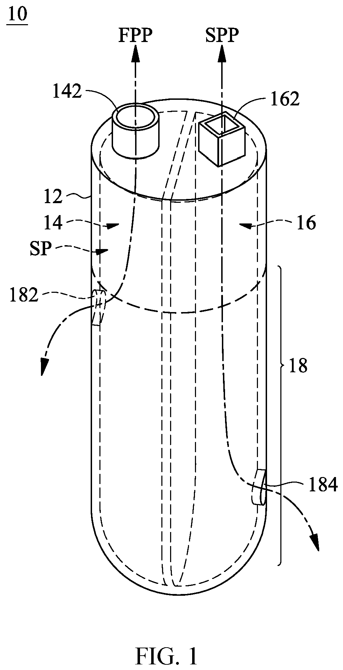

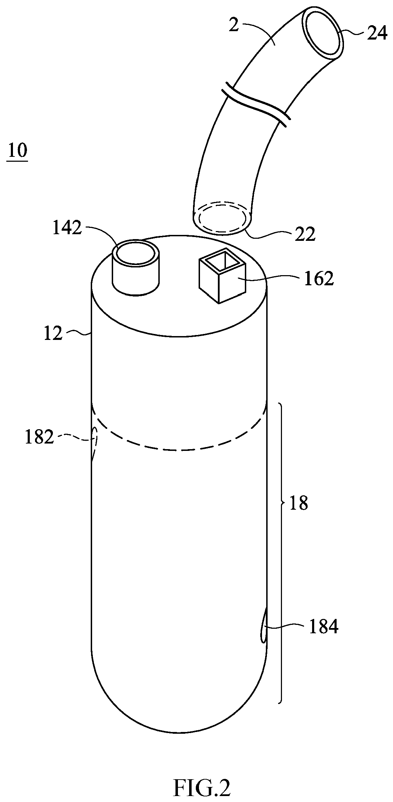

Please refer to FIG.1, where it is a structure diagram of a first embodiment of a multi-tube adapter of the present invention. In FIG. 1, the multi-tube adapter 10 may be connected to one or a plurality of outer tubes 2 (with reference to FIG.2) for application to the human digestive system (e.g. esophagus, stomach, intestine, etc.) and respiratory system (e.g. throat, trachea, bronchus, lungs, etc.). An end 22 of the outer tube 2 is connected to the multi-tube adapter 10 while the other end 24 may be selectively connected to another equipment such as a gas cylinder, an external breathing equipment, a negative pressure equipment or a spray equipment, etc. Notably, the outer tube 2 may be freely assembled and disassembled, or be omitted. For example, if the multi-tube adapter 10 needs to obtain oxygen from a gas cylinder, the multi-tube adapter 10 and the gas cylinder may be connected through the outer tube 2 for supply of oxygen; if the function of the multi-tube adapter 10 is to provide nebulized medicine, the medicine may be directly received without the use of the outer tube 2.

Back to FIG. 1, the multi-tube adapter 10 includes a housing 12.

The housing 12 forms an accommodating space SP which includes a first chamber 14 and a second chamber 16. Herein, the shape of the housing 12 is tubular for illustration purpose, nevertheless, the housing 12 may be of any shape, size, material, etc. without any constraint in the present invention. The number of chambers is two for illustration purpose, whereas in other embodiments, the number of chambers may be increased to three, four or more. Notably, for illustration, the first chamber 14 is designated to supply gas (or powder) while the second chamber 16 is designated to supply liquid.

Furthermore, the first chamber 14 and the second chamber 16 are not connected to each other, herein the formation method of the first chamber 14 and the second chamber 16 is not limited, for example, the formation method may be all-in-one or adding a partition board (not shown) in the accommodating space (SP) to segment the first chamber 14 and the second chamber 16. In one embodiment, the dimensions of the first chamber 14 are smaller than or equal to those of the second chamber 16, or the dimensions of the second chamber 16 are smaller than or equal to those of the first chamber 14.

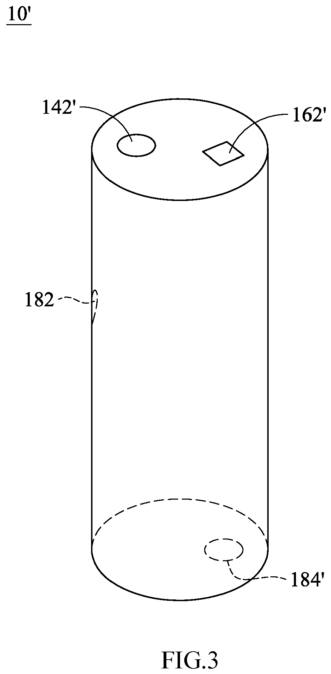

The first chamber 14 has a first connection port 142 while the second chamber 16 has a second connection port 162. Herein, as an example for illustration, the first connection port 142 is of cylindrical shape whereas the second connection port 162 is of rhombic shape, but other embodiments are not limited to the aforesaid shapes. The first connection port 142 and the second connection port 162 may be arranged to have different shapes to achieve the foolproof (or called anti-misplacement) purpose. Moreover, the first connection port 142 and the second connection port 162 have a protruding shape, while in another embodiment, with reference to FIG.3 of a second embodiment of a multitube adapter 10' wherein the first connection port 142' and the second connection port 162' thereof are flat openings.

Back to FIG. 1, the first chamber 14 and the second chamber are connected to an extension 18 having a first opening 182 and a second opening 184, wherein the diameter of the first opening 182 is smaller than or equal to the diameter of the second opening 184; alternatively, the diameter of the second opening 184 is smaller than or equal to the diameter of the first opening 182.

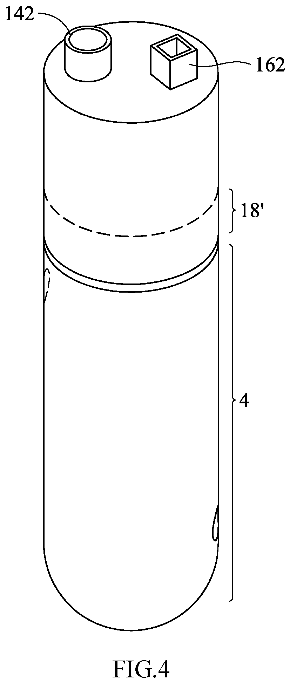

Herein, the first chamber 14 is connected to the first opening 182 and the second chamber 16 is connected to the second opening 184. Furthermore, the first opening 182 may be corresponded to the trachea (not shown) and the second opening 184 may be corresponded to the stomach (not shown). In this embodiment, the extension 18 can directly act as an inner tube, or the extension 18 further connects to another external inner tube 4 (as shown in FIG.4). Note that the interior of the external inner tube 4 needs to form corresponding space in accordance with the number of openings, that is, the interior of the external inner tube 4 has the extending characteristic of the chamber, and the spaces inside of the external inner tube 4 also have the characteristic of the corresponding chambers in that they are not connected with one another.

Referring back to FIG.3, the location of the first opening 182 and the second opening 184 are not limited in the present disclosure; the first opening 182 and the second opening 184 may be provided on the sides of the extension 18 as in FIG. 1, while the second opening 184' may also be provided at the bottom of the extension 18 as in FIG.3. In addition, the length and shape of the extension 18 are also not limited. In FIG.4, as the extension 18' is able to connect to the external inner tube 4, extension 18' does not need to be too long. Hence, the length of the extension 18 in FIG.1 is conspicuously larger than the length of the extension 18' in FIG.4.

Back to FIG. 1, the first opening 182 is connected to the first chamber 14 while the second opening 184 is connected to the second chamber 16, wherein the first connection port 142 connects to the first chamber 14 and the first opening 182, enabling the first connection port 142 to further establish a first supply path FPP with, for example, the respiratory system, through the first chamber 14 and the first opening 182; the second connection port 162 is connected to the second chamber 16 and the second opening 184, enabling the second connection port 162 to further establish a second supply path SPP with, for example, the digestive system through the second chamber 16 and the second opening 184.

According to different application scenarios, the first supply path FPP and the second supply path SPP have primarily several configurations, for example: a first configuration in which the first supply path serves to draw secretions such as sputum from the throat through negative pressure to maintain smooth breathing; a second configuration in which the first supply path FPP serves to draw in external medicine powder, medicine spray, oxygen or water vapor spray and supply same to the throat for treatment; a third configuration in which the second supply path acts similarly to a nasogastric tube to provide feeding from outside; a fourth configuration in which the third configuration, if in use, is employed simultaneously with the first configuration or the second configuration. Besides the aforementioned four configurations, other unmentioned configurations are further included.

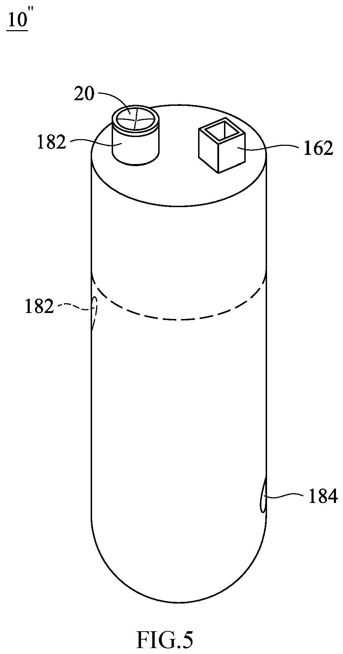

Please refer to FIG.5, a structure diagram of a fourth embodiment of the multitube adapter of the present disclosure. In FIG.5, the multi-tube adapter 10" further includes an open-close component 20 in addition to the housing 12 of the first embodiment.

The open-close component 20 is disposed at the first chamber 14. The open-close component 20 selectively connects to the first connection port 142 and the first opening 182. The first connection port 142 based on the open-close component 20 selectively connects to the first chamber 14 and the first opening 182, enabling the first connection port 142 to further establish the first supply path FPP through the first chamber 14 and the first opening 182.

For example, the open-close component 20 further includes a baffle (not shown) and an elastomer (not shown). The baffle is coupled with the elastomer. One end of the baffle is disposed at the side wall of the first connection port 142 while the other end of the baffle is placed against the other side wall of the first connection port 142, so as to connect the first connection port 142 and the first chamber 14 due to the deformation of the elastomer when an external force is applied to the baffle.

Moreover, the open-close component 20 may have a passive opening structure of which the configuration patterns are: (1) in the natural situation, the open-close component 20 closes the first supply path FPP or the second supply path SPP; and (2) when an outer tube is inserted, for example, the open-close component 20 establishes the first supply path FPP or the second supply path SPP.

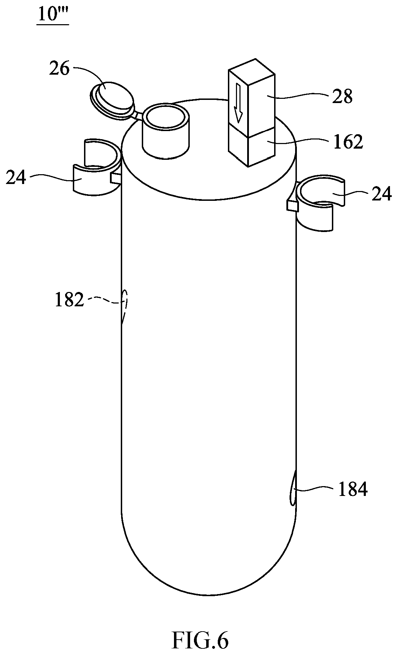

Please refer to FIG.6, a structure diagram of a fifth embodiment of the multitube adapter of the present disclosure. In FIG.6, the multi-tube adapter 10" further includes a fastener 24, a closing component 26 and an indicator 28 in addition to the housing 12 of the first embodiment.

The fastener 24 is disposed on the housing 12 for fixing the housing 12 onto a human body or fixing an outer tube, wherein the fastener 24, for example, may be configured to be a buckle or a c-ring. In the configuration of fixing onto the human body, the multi-tube adapter 10‴ may be fixed to the head via a strap in combination with a buckle; in the fixing the outer tube configuration, the outer tube may be snapped on via the c-ring.

The closing component 26 is couple with the connection port 142 (or the second connection port 162) for selectively closing the first supply path FPP and the second supply path SPP; for example, the close component 26 may be a lid, a cover or a valve, etc., and the lid shall be taken as an example for illustration.

The indicator 28 is disposed at the opening of the first connection port 142 (or the second connection port 162), capable of indicating the supply direction of, for example, the first supply path FPP and the second supply path SPP, and the form thereof may be a sleeve.

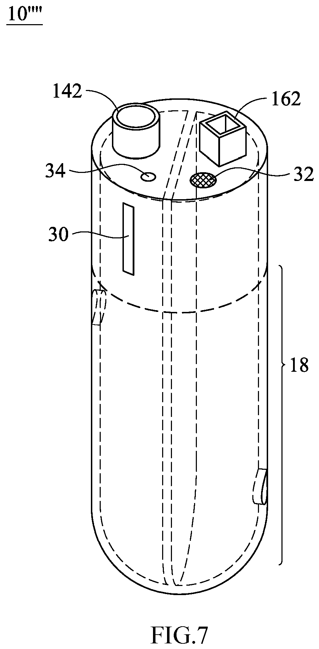

Please refer to FIG.7, a structure diagram of a sixth embodiment of the multitube adapter of the present disclosure. In FIG.7, the multi-tube adapter 10ʺʺ further includes a sensor module 30, an output module 32 and an input module 34 in addition to the housing 12 of the first embodiment.

The sensor module 30 is disposed on the housing 12 for detecting the statuses of the first connection port, the second connection port, the first chamber, the second chamber, the first opening, the second opening, the first supply path, the second supply path, wherein the statuses include physical quantities such as gas flow, liquid flow, light source, sound source, temperature, humidity, wind power, oxygen concentration, carbon dioxide concentration, PH value, height, etc. According to the detection of the aforesaid physical quantities, airflow status, feeding counts, feeding time, etc. may be obtained through calculation by an application (APP). The output module is disposed on the housing, the output module emitting light, outputting thermal energy, making sounds, generating micro currents, etc.

The output module 32 is disposed on the housing 12. The output module 32 is capable of emitting light, outputting thermal energy, making sounds, generating micro currents, generating vibrations, and may be, for example, an LED component, an electrothermal coupling component, a speaker, a battery, a motor, etc.

The input module 34 is disposed on the housing 12. The input module 34 captures sound, video and may be a microphone, a photographic lens, etc.

The present invention is disclosed in the abovementioned description by several preferred embodiments, but it is supposed to be comprehended by those who are skilled in the art that the embodiments are used only to illustrate the present invention rather than restrict the scope of the present invention. Therefore, what is claimed in the present invention shall be subject to the claims.

## Claims

1. A multi-tube adapter connected to a plurality of outer tubes (2) for application to human digestive system and respiratory system, the multi-tube adapter being inserted through the nasal cavity, throat and esophagus into the stomach, and including:
a housing (12) forming an accommodating space (SP) having a first chamber (14) and a second chamber (16) , the first chamber (14) and the second chamber (16) being not connected to each other, wherein the first chamber (14) has a first connection port (142) and the second chamber (16) has a second connection port (162), and the first chamber (14) and the second chamber (16) have an extension (18) having a first opening (182) adjacent to the respiratory system and a second opening (184) adjacent to stomach, the first opening (182) being connected to the first chamber (14) and the second chamber (16) being connected to the second opening (184);
wherein the first connection port (142) is connected to the first chamber (14) and the first opening (182), enabling the first connection port (142) to further establish a first supply path (FPP) with the respiratory system through the first chamber (14) and the first opening (182), and the second connection port (162) is connected to the second chamber (16) and the second opening (184), enabling the second connection port (162) to further establish a second supply path (SPP) with the digestive system through the second chamber (16) and the second opening (184), **characterized in that**
the multi-tube adapter further includes a closing component (26) coupled to at least one of the first connection port (142) and the second connection port (162) for selectively closing the first supply path (FPP) and the second supply path (SPP).

2. The multi-tube adapter according to claim 1, further including an open-close component (20) disposed at the first chamber(14), the open-close component (20) being selectively to the first connection port (142) and the first opening (182), the first connection port (142) being connected to the first chamber (14) and the first opening (182) in accordance with the open-close component (20), enabling the first connection port (142) to further establish the first supply path (FPP) with the respiratory system through the first chamber (14) and the first opening (182).

3. The multi-tube adapter according to claim 1, further including a fastener (24) disposed at the housing (12) for fixing the housing (12) onto a human body or fixing outer tubes (2) thereon.

4. The multi-tube adapter according to claim 1, further including an indicator (28) disposed at an opening of at least one of the first connection port (142) and the second connection port (162) for indicating the first supply path (FPP) and the second supply path (SPP).

5. The multi-tube adapter according to claim 1, wherein at least one of the first connection port (142) and the second connection port (162) has a foolproof mechanism.

6. The multi-tube adapter according to claim 1, further including a sensor module (30) disposed at the accommodating space (SP) for detecting the statuses of at least one of the first connection port, (142) the second connection port (162), the first chamber (14), the second chamber (16), the first opening (182), the second opening (184), the first supply path (FPP), the second supply path (SPP), wherein the statuses include at least one of the physical quantities such as gas flow, liquid flow, oxygen concentration, carbon dioxide concentration, PH value, light source, sound source, temperature, humidity, wind power and height.

7. The multi-tube adapter according to claim 1, further including an output module (32) disposed at the housing (12), the output module (32) being capable of at least one of emitting light, outputting thermal energy, making sounds, generating micro currents, generating vibrations.

8. The multi-tube adapter according to claim 1, further including an input module (34) disposed at the housing (12), the input module (34) capturing at least one of sound and video.

9. The multi-tube adapter according to claim 1, wherein the second opening (184) of the extension (18) is located at the bottom or on the sides of the extension (18), and the first opening (182) of the extension (18) is located on the sides of the extension (18).

## Patentansprüche

1. Mehrschlauchadapter, der mit einer Vielzahl von Außenschläuchen (2) verbunden ist, zur Anwendung im menschlichen Verdauungssystem und Atmungssystem, wobei der Mehrschlauchadapter durch die Nasenhöhle, den Rachen und die Speiseröhre in den Magen eingeführt ist und umfasst:
ein Gehäuse (12), das einen Aufnahmeraum (SP) mit einer ersten Kammer (14) und einer zweiten Kammer (16) bildet, wobei die erste Kammer (14) und die zweite Kammer (16) nicht miteinander verbunden sind, wobei die erste Kammer (14) einen ersten Verbindungsanschluss (142) aufweist und die zweite Kammer (16) einen zweiten Verbindungsanschluss (162) aufweist und die erste Kammer (14) und die zweite Kammer (16) eine Verlängerung (18) mit einer ersten Öffnung (182), die an das Atmungssystem angrenzt, und einer zweiten Öffnung (184), die an den Magen angrenzt, aufweisen, wobei die erste Öffnung (182) mit der ersten Kammer (14) verbunden ist und die zweite Kammer (16) mit der zweiten Öffnung (184) verbunden ist;
wobei der erste Verbindungsanschluss (142) mit der ersten Kammer (14) und der ersten Öffnung (182) verbunden ist, wodurch der erste Verbindungsanschluss (142) befähigt wird, weiter einen ersten Versorgungspfad (FPP) mit dem Atmungssystem durch die erste Kammer (14) und die erste Öffnung (182) herzustellen, und der zweite Verbindungsanschluss (162) ist mit der zweiten Kammer (16) und der zweiten Öffnung (184) verbunden, wodurch der zweite Verbindungsanschluss (162) befähigt wird, weiter einen zweiten Versorgungspfad (SPP) mit dem Verdauungssystem durch die zweite Kammer (16) und die zweite Öffnung (184) herzustellen, **dadurch gekennzeichnet, dass**
der Mehrschlauchadapter ferner eine Verschlusskomponente (26) umfasst, die mit zumindest einem von dem ersten Verbindungsanschluss (142) und dem zweiten Verbindungsanschluss (162) gekoppelt ist, um den ersten Versorgungspfad (FPP) und den zweiten Versorgungspfad (SPP) selektiv zu verschließen.

2. Mehrschlauchadapter nach Anspruch 1, welcher ferner eine Öffnungs-Schließ-Komponente (20) umfasst, die an der ersten Kammer (14) angeordnet ist, wobei die Öffnungs-Schließ-Komponente (20) selektiv für den ersten Verbindungsanschluss (142) und die erste Öffnung (182) ist, wobei der erste Verbindungsanschluss (142) entsprechend der Öffnungs-Schließ-Komponente (20) mit der ersten Kammer (14) und der ersten Öffnung (182) verbunden ist, damit der erste Verbindungsanschluss (142) in der Lage ist, weiter den ersten Versorgungspfad (FPP) mit dem Atmungssystem durch die erste Kammer (14) und die erste Öffnung (182) herzustellen.

3. Mehrschlauchadapter nach Anspruch 1, welcher ferner ein Befestigungsmittel (24) umfasst, das an dem Gehäuse (12) angeordnet ist, um das Gehäuse (12) an einem menschlichen Körper zu befestigen oder um Außenschläuche (2) daran zu befestigen.

4. Mehrschlauchadapter nach Anspruch 1, welcher ferner eine Anzeige (28) umfasst, die an einer Öffnung von mindestens einem von dem ersten Verbindungsanschluss (142) und dem zweiten Verbindungsanschluss (162) angeordnet ist, um den ersten Versorgungspfad (FPP) und den zweiten Versorgungspfad (SPP) kenntlich zu machen.

5. Mehrschlauchadapter nach Anspruch 1, bei welchem zumindest einer von dem ersten Verbindungsanschluss (142) und dem zweiten Verbindungsanschluss (162) einen Betriebssicherheitsmechanismus hat.

6. Mehrschlauchadapter nach Anspruch 1, welcher ferner ein Sensormodul (30) umfasst, das an dem Aufnahmeraum (SP) angeordnet ist, um die Zustände von zumindest einem von dem ersten Verbindungsanschluss (142), dem zweiten Verbindungsanschluss (162), der ersten Kammer (14), der zweiten Kammer (16), der ersten Öffnung (182), der zweiten Öffnung (184), des ersten Versorgungspfades (FPP), des zweiten Versorgungspfades (SPP) zu erfassen, wobei die Zustände mindestens eine der physikalischen Größen, wie Gasdurchfluss, Flüssigkeitsdurchfluss, Sauerstoffkonzentration, Kohlendioxidkonzentration, PH-Wert, Lichtquelle, Schallquelle, Temperatur, Feuchtigkeit, Windstärke und Höhe, umfassen.

7. Mehrschlauchadapter nach Anspruch 1, welcher ferner ein Ausgabemodul (32) umfasst, das an dem Gehäuse (12) angeordnet ist, wobei das Ausgabemodul (32) fähig ist zu mindestens einem von Emission von Licht, Abgabe von Wärmeenergie, Machen von Geräuschen, Erzeugung von Mikroströmen, Erzeugung von Vibrationen.

8. Mehrschlauchadapter nach Anspruch 1, welcher ferner ein Eingabemodul (34) umfasst, das an dem Gehäuse (12) angeordnet ist, wobei das Eingabemodul (34) mindestens eines von Ton und Video aufzeichnet.

9. Mehrschlauchadapter nach Anspruch 1, bei welchem sich die zweite Öffnung (184) der Verlängerung (18) am Boden oder an den Seiten der Verlängerung (18) befindet, und sich die erste Öffnung (182) der Verlängerung (18) an den Seiten der Verlängerung (18) befindet.

## Revendications

1. Adaptateur multitube connecté à une pluralité de tubes externes (2) pour une application au système digestif et au système respiratoire humains, l'adaptateur multitube étant inséré à travers la cavité nasale, la gorge et l'œsophage jusque dans l'estomac, et incluant :
un boîtier (12) formant un espace de réception (SP) présentant une première chambre (14) et une seconde chambre (16), la première chambre (14) et la seconde chambre (16) n'étant pas connectées l'une à l'autre, dans lequel la première chambre (14) présente un premier orifice de connexion (142) et la seconde chambre (16) présente un second orifice de connexion (162), et la première chambre (14) et la seconde chambre (16) présentent une extension (18) présentant une première ouverture (182) adjacente au système respiratoire et une seconde ouverture (184) adjacente à l'estomac, la première ouverture (182) étant connectée à la première chambre (14) et la seconde chambre (16) étant connectée à la seconde ouverture (184) ;
dans lequel le premier orifice de connexion (142) est connecté à la première chambre (14) et à la première ouverture (182), permettant au premier orifice de connexion (142) d'établir en outre un premier trajet d'alimentation (FPP) avec le système respiratoire à travers la première chambre (14) et la première ouverture (182), et le second orifice de connexion (162) est connecté à la seconde chambre (16) et à la seconde ouverture (184), permettant au second orifice de connexion (162) d'établir en outre un second trajet d'alimentation (SPP) avec le système digestif à travers la seconde chambre (16) et la seconde ouverture (184),
**caractérisé en ce que**
l'adaptateur multitube inclut en outre un composant de fermeture (26) couplé à au moins l'un du premier orifice de connexion (142) et du second orifice de connexion (162) pour fermer sélectivement le premier trajet d'alimentation (FPP) et le second trajet d'alimentation (SPP).

2. Adaptateur multitube selon la revendication 1, incluant en outre un composant d'ouverture-fermeture (20) disposé au niveau de la première chambre (14), le composant d'ouverture-fermeture (20) étant connecté sélectivement au premier orifice de connexion (142) et à la première ouverture (182), le premier orifice de connexion (142) étant connecté à la première chambre (14) et à la première ouverture (182) selon le composant d'ouverture-fermeture (20), permettant au premier orifice de connexion (142) d'établir en outre le premier trajet d'alimentation (FPP) avec le système respiratoire à travers la première chambre (14) et la première ouverture (182).

3. Adaptateur multitube selon la revendication 1, incluant en outre une fixation (24) disposée au niveau du boîtier (12) pour fixer le boîtier (12) sur un corps humain ou pour fixer des tubes externes (2) sur celui-ci.

4. Adaptateur multitube selon la revendication 1, incluant en outre un indicateur (28) disposé au niveau d'une ouverture d'au moins l'un du premier orifice de connexion (142) et du second orifice de connexion (162) pour indiquer le premier trajet d'alimentation (FPP) et le second trajet d'alimentation (SPP).

5. Adaptateur multitube selon la revendication 1, dans lequel au moins l'un du premier orifice de connexion (142) et du second orifice de connexion (162) présente un mécanisme infaillible.

6. Adaptateur multitube selon la revendication 1, incluant en outre un module de capteur (30) disposé au niveau de l'espace de réception (SP) pour détecter les états d'au moins l'un du premier orifice de connexion (142), du second orifice de connexion (162), de la première chambre (14), de la seconde chambre (16), de la première ouverture (182), de la seconde ouverture (184), du premier trajet d'alimentation (FPP), du second trajet d'alimentation (SPP), dans lequel les états incluent au moins l'une des quantités physiques telles que le débit de gaz, le débit de liquide, la concentration en oxygène, la concentration en dioxyde de carbone, la valeur de pH, la source de lumière, la source de son, la température, l'humidité, la puissance du vent et la hauteur.

7. Adaptateur multitube selon la revendication 1, incluant en outre un module de sortie (32) disposé au niveau du boîtier (12), le module de sortie (32) étant capable d'au moins l'une parmi l'émission de lumière, la délivrance en sortie d'énergie thermique, la production de sons, la génération de micro-courants, la génération de vibrations.

8. Adaptateur multitube selon la revendication 1, incluant en outre un module d'entrée (34) disposé au niveau du boîtier (12), le module d'entrée (34) capturant au moins l'un d'un son et d'une vidéo.

9. Adaptateur multitube selon la revendication 1, dans lequel la seconde ouverture (184) de l'extension (18) est située en bas ou sur les côtés de l'extension (18), et la première ouverture (182) de l'extension (18) est située sur les côtés de l'extension (18).
